(19) Europäisches Patentamt
European Patent Office
Office européen des brevets

(11) EP 3 519 590 B1

(12) **EUROPEAN PATENT SPECIFICATION**

(45) Date of publication and mention of the grant of the patent:
**04.11.2020 Bulletin 2020/45**

(21) Application number: **17791158.3**

(22) Date of filing: **29.09.2017**

(51) Int Cl.:
***C12Q 1/68*** *(2018.01)*

(86) International application number:
**PCT/IB2017/055998**

(87) International publication number:
**WO 2018/060942 (05.04.2018 Gazette 2018/14)**

(54) **BIO INFORMATIC METHOD FOR EVALUATING THE RISK FOR ONSET OF AGE RELATED MACULAR DEGENERATION**

BIOINFORMATISCHES VERFAHREN ZUR VORHERSAGE DES RISIKOS DES ALTERSBEDINGTER MACULADEGENERATIONS

PROCEDE BIO-INFORMATIQUE DESTINE A IDENTIFIER LES RISQUE D'APPARITION DE LA DÉGÉNÉRATION MACULAIRE LIÉE À L'ÂGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.09.2016 IT 201600098461**

(43) Date of publication of application:
**07.08.2019 Bulletin 2019/32**

(73) Proprietor: **SIFI S.p.A.**
**95025 Aci Sant'Antonio (CT) (IT)**

(72) Inventors:
- **SPATA, Massimo**
**95123 Catania (IT)**
- **BLANCO, Anna Rita**
**95024 Acireale (IT)**
- **CONOCI, Sabrina**
**95030 Canalicchio (IT)**
- **CURATOLO, Maria Cristina**
**95025 Aci Sant'Antonio (IT)**

(74) Representative: **Torti, Carlo Maria Emilio et al**
**Notarbartolo & Gervasi S.p.A.**
**Viale Achille Papa, 30**
**20149 Milano (IT)**

(56) References cited:
- **JOHANNA M. SEDDON ET AL: "Risk Prediction for Progression of Macular Degeneration: 10 Common and Rare Genetic Variants, Demographic, Environmental, and Macular Covariates", INVESTIGATIVE OPTHALMOLOGY & VISUAL SCIENCE, vol. 56, no. 4, 10 April 2015 (2015-04-10) , page 2192, XP055390681, US ISSN: 1552-5783, DOI: 10.1167/iovs.14-15841**
- **JOHANNA M. SEDDON ET AL: "Three New Genetic Loci (R1210C in CFH, Variants in COL8A1 and RAD51B) Are Independently Related to Progression to Advanced Macular Degeneration", PLOS ONE, vol. 9, no. 1, 31 January 2014 (2014-01-31), page e87047, XP055390659, DOI: 10.1371/journal.pone.0087047**
- **SWAROOP ANAND ET AL: "Unraveling a multifactorial late-onset disease: from genetic susceptibility to disease mechanisms for age-related macular degeneration", ANNUAL REVIEW OF GENOMICS AND HUMAN GENETICS, ANNUAL REVIEWS, US, vol. 10, 1 September 2009 (2009-09-01), pages 19-43, XP002584686, ISSN: 1527-8204, DOI: 10.1146/ANNUREV.GENOM.9.081307.164350 [retrieved on 2009-04-28]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 519 590 B1

## Description

The present invention relates to an advanced bio-computer analysis method for evaluating the risk of the onset of age-related macular degeneration (AMD). In particular, the present invention relates to such a method which correctly evaluates the impact of genetic factors combined with environmental factors. In detail, the present invention relates to such a method which allows to estimate the genetic AMD risk more accurately than the methods known in the art.

### Background art

Apparatuses and methods related to the subject of the present invention are known in the art.

In particular, a test from SOOFT (hereinafter referred to as "SOOFT test") is available on the market for evaluating the risk level of AMD onset in a subject based on genetic and environmental factors. DNA is obtained by means of a common oral swab. The operation is very simple, fast and completely painless and consists in using a sterile, disposable, soft brush which is rubbed for a few seconds against the inner wall of the subject's cheeks. Such an operation allows to obtain small amounts of cells containing the subject's DNA from the oral mucosa, which DNA will be extracted and subjected to genetic testing. At present, genetic testing is carried out in a specialized laboratory by qualified personnel and it is expensive.

This SOOFT test evaluates the risk of macular degeneration based on two different series of genetic and individual parameters. In particular, the SOOFT test uses, as genetic parameters, 7 polymorphisms divided on 5 genes: ARMS2 (Age-Related Maculopathy Susceptibility 2) rs10490924; CFH (Complement Factor H) rs1061170, rs1410996 and rs403846; C3 (Complement component 3) rs2230199; CFB (Complement factor B) rs641153; C2 (Complement Component 2) rs9332739, whereas ,as individual parameters, uses the following 6 parameters: age, gender, body mass index; smoker; family history, advanced AMD in one eye. Therefore, the current SOOFT test offers a statistical test using a small set of input parameters (only 5 genes and 6 environmental factors).

Therefore, it is the object of the present invention to provide a more reliable method for determining the evaluation of the risk of the age-related macular degeneration onset.

Accordingly, in this context, the present invention is conceived to meet the need to find an alternative method capable of providing for a greater accuracy in the input parameters, in particular a dynamic set of parameters which can be adapted to a wider resolution of problems of the same category.

### Summary of the invention.

In a first aspect, the present invention relates to a method according to claim 1.

In fact, the present invention arises from the general consideration that the above-mentioned technical problem can be effectively and reliably solved by means of a method for estimating the risk factor (HR) for the onset of macular degeneration in a subject, which method comprises the evaluation of a first series of genetic parameters which comprise at least the genetic loci R1210C in CFH, variants in COL8A1 and RAD51B, and the evaluation of a second series of environmental, individual and/or clinical parameters. Such a method is characterized in that said risk factor (HR) is calculated by assigning a greater importance to said evaluation of the first series of genetic parameters with respect to said evaluation of the second series of environmental, individual and/or clinical parameters.

The method of the present invention thus allows to estimate the genetic risk of AMD more accurately than the methods known in the art.

According to an embodiment, said risk factor (HR) is calculated by assigning an importance in a range of 51%-70% to the evaluation of said first series of genetic parameters and an importance in a range of 30%-49% to the evaluation of said second series of environmental, individual and/or clinical parameters.

According to an embodiment, said risk factor (HR) is calculated by assigning an importance of about 60% to the evaluation of said first series of genetic parameters and an importance of about 40% to the evaluation of said second series of environmental, individual and/or clinical parameters.

Therefore, giving a greater importance to the evaluation of the first series of genetic parameters, an even more accurate estimate is obtained.

According to an embodiment, said first series of genetic parameters further comprises at least the following additional genes: ARMS2 (Age-Related Maculopathy Susceptibility 2), CFH (Complement Factor H), C3 (Complement component 3); CFB (Complement factor B) and C2 (Complement Component 2). According to an embodiment, said first series of genetic parameters further comprises at least the following polymorphisms of said additional genes: polymorphism rs10490924 of ARMS2; polymorphisms of rs1061170, rs1410996 and rs403846 of CFH; polymorphism rs2230199 of C3; polymorphism rs641153 of CFB, and polymorphism rs9332739 of C2.

According to an embodiment, said environmental parameters of said second series are selected from the group comprising at least those related to whether the subject is a smoker, and those related to an oxidant-based diet.

According to an embodiment, said clinical parameters of said second series are selected from the group related to cardiovascular problems, hypertension, diabetes, family history, AMD diagnosed in one eye, and previous cataract surgeries. According to an embodiment, said individual parameters of said second series are selected from the group related to age, gender, and body mass index.

The risk factor HR represents the risk ratio in the survival analysis and it is the effect of an explanatory variable about the danger or risk of an event.

According to an embodiment, the evaluation of said first series of genetic parameters is calculated by averaging the individual evaluations obtained for each of said genetic parameters, each of said individual evaluations being obtained based on the ratio of the events observed in a period of time (Δt) to the number of possible events at risk at the beginning of said period of time (Δt), when said period of time (Δt) tends to zero.

In particular, each evaluation h as a function of time t is defined by the following formula (I):

$$h(t) = \lim_{\Delta t \to 0} \frac{E(t)/N(t)}{\Delta t} \qquad \text{(I)}$$

where *N(t)* is the number of events at risk at the beginning of an interval of time t and E(t) represents the events observed at time t, whereas Δ*t* represents the period of time taken into account.

Similarly, according to an embodiment, the evaluation of said second series of environmental, individual and/or clinical parameters is calculated by averaging the individual evaluations obtained for each of said environmental, individual and/or clinical parameters, each of said individual evaluations being obtained based on the ratio of the events observed in a period of time (Δt) to the number of possible events at risk at the beginning of said period of time (Δt) when said period of time (Δt) tends to zero.

According to an embodiment, said risk factor (HR) is calculated based on said weighted averages of the evaluations of the first and second series of evaluations.

According to an embodiment, said risk factor (HR) is evaluated as high, medium or low, according to whether, by means of the method of the present invention, it is respectively greater than given predetermined threshold values t3, t2 or t1, with t3 > t2 > t1.

The DNA on which the AMD genetic test is performed can be extracted from any cell type.

According to an embodiment, the subject's blood can be used to determine the DNA; thereby, the withdrawal is very simple and a few blood drops are enough to achieve reliable and definitely more reproducible results.

According to another embodiment, buccal epithelial cells may be used to determine the DNA, which are harvested, for example, by means of a common oral swab. However, in the latter case, the compression used by the operator with the swab may weigh on the amount of harvested cells.

According to an embodiment, the blood (or swab) can be processed directly at the medical laboratory where the sample was withdrawn, using a simple lysis solution loaded on a silicon chip-based platform, such as the platform Q3 RT-PCR available from the Applicant of the present patent application.

The risk factor estimation method of the present invention can thus be conducted directly at the medical laboratory by non-specialized personnel and the response will be quick and easy to be interpreted.

The genetic results will then be integrated with the personal, clinical, and environmental data that the practitioner can easily obtain from the patient by filling in a short questionnaire.

Any further embodiments of the present invention are specified in the claims.

The present invention is disclosed below in greater detail by means of a detailed description of the exemplary and non-limiting embodiment.

## Detailed description

A test was performed to determine the risk for the onset of age-related macular degeneration (AMD) in a human subject.

The DNA on which the genetic test was performed was determined by using the subject's blood. The blood (or swab) was processed directly at the medical laboratory where the withdrawal was carried out, by using a simple lysis solution loaded on a silicon chip-based platform, such as the Q3RT-PCR platform available from the Applicant of the present patent application.

The risk factor estimation method of the present invention was conducted directly at the medical laboratory by non-specialized personnel and the response was quick and easy to be interpreted.

Predetermined threshold values t1, t2 and t3 were set, equal to $0 =< t_1 <= 25\%$, $25\% < t_2 <= 50\%$, $t_3 > 50\%$, respectively.

Such risk threshold values were determined as a function of the current standards used by the scientific community as risk parameters.

A first series of genetic parameters was analyzed, comprising the genetic loci R1210C in CFH, variants in COL8A1 and RAD51B, and polymorphisms rs10490924 of ARMS2 (Age-Related Maculopathy Susceptibility 2), polymorphisms rs1061170, rs1410996 and rs403846 of CFH (Complement Factor H), polymorphism rs2230199 of C3 (Complement component 3), polymorphism rs641153 of CFB (Complement factor B), and polymorphism rs9332739 of C2 (Complement Component 2), for a total of 9 polymorphisms divided into 5 genes.

For each of such genetic parameters, a risk factor component was calculated as follows.

Given a sample of $m$ individuals $S = \{S_1, S_2, ..., S_m\}$, $D_p$ and $D_n$ being the classes of AMD positive and negative individuals, respectively.

Given the set of risk factors HR = $\{h_1, h_2, ..., h_n\}$ for $\forall S_i \in S$, the estimated weights based on the statistical sample for each $h_i$ can be determined

$$W = \{w_1, w_2, \ldots, w_n\}: w_1 + w_2 + \ldots + w_n \leq 1$$

With the $h_i$ factors provided on the sample of AMD positive and negative patients, $x_h[i]$ are calculated using the formula:

$$x_h[i] = \sup(h[i], Dp) - \sup(h[i], Dn)$$

With the previous calculated values of $x_i$ and $w_i$, the geometric weighed average p of all such individual evaluations was then calculated using the following formula (a):

$$p = exp\left(\frac{\sum_{i=1}^{n} w_i \ln x_i}{\sum_{i=1}^{n} w_i}\right) \text{(a)}$$

If a database containing the data related to the sampled patients is available, this information can be automatically extracted from the database through a simple search and calculated in real time by applying formula (a).

The genetic results were then integrated with the personal and clinical data and with the environmental factors $h_i$ that the practitioner had obtained from the patient by filling in a short questionnaire.

A second series of environmental, individual and/or clinical parameters $h_i$ was thus analyzed.

The environmental parameters taken into account were those related to whether the subject is a smoker ("smoking"), and those related to an oxidant-based diet ("oxidant"). The clinical parameters taken into account were those related to cardiovascular problems ("cardiovascular"), hypertension ("hypertension"), diabetes ("diabetes"), family history ("hist"), AMD diagnosed in one eye ("AMD"), and previous cataract surgeries ("catarat"). Finally, the individual parameters taken into account were age ("age"), gender ("gender"), body mass index ("obesity"), for a total of 11 factors related to environmental, individual and/or clinical parameters.

Similarly to the above with reference to the evaluation of the genetic parameters, for each of these environmental, individual and/or clinical parameters $h_i$, a component $w_i$ of the risk factor was calculated, the sum of such components being not higher than 1.

For example, a weight factor $w_i$ was assigned to the age-related risk factor as follows:

$w_i$ = 0.0 if age is a value from 40 to 49
$w_i$ =0.002 if age is a value from 50 to 59
$w_i$ = 0.003 if age is a value from 60 to 69
$w_i$ = 0.015 if age is a value from 70 to 79
$w_i$= 0.035 if age is a value higher than 80.

The value of p was then calculated with each of the previously listed risk factors $h_i$ (environmental parameters and genetic factors), thus obtaining:

p = p_age + p_gender + p_obesity + p_smoking + p_oxydant + p_cardiovascular + p_hipertension + p_diabetes + p_amd + p_cataract + p_hist

The value of p_genetic_factors was calculated for the genetic parameters, equal to the sum of p related to the following genotype information: CFH (rs1061170), CFH (rs1410996), HTRA1 (rs10490924), C2 (rs9332739), CFB (rs641153), C3 (rs2230199), COL8A1 (rs13095226), CFH (rs121913059), RAD51 B (rs8017304).
A weighted average of all these individual evaluations was then made.
An importance of 60% was assigned to the weighted average related to the genetic parameters, whereas an importance of 40% was assigned to the weighted average of environmental, individual and/or clinical parameters.
Finally, the risk percentage was calculated according to the formula:

$$risk = (1 - y^p) * 100$$

where y is the number of years on which the risk calculation projection is to be carried out (0 to 10 years), for example:

y = 0.98 for two years
y = 0.97 for three years
y = 0.96 for four years
y = 0.95 for five years
y = 0.94 for six years
y = 0.93 for seven years
y = 0.92 for eight years
y = 0.91 for nine years
y = 0.90 for ten years

Given $t_3 = 50$, $t_2$ from 25 to 50, and $t_1$ from 0 to 25, the risk will be high if the variable "risk" is higher than $t_3$, medium if "risk" is in the range of $t_2$, and low if "risk" is in the range of $t_1$.
The following algorithm can generally be applied:

Input: for each of the m subjects $s_1,..,s_m$ of the set S, the n parameters $h_1,..,h_n$ of the risk factors H, the n relative weights $w_1,..,w_n$ and the threshold values $t_1$, $t_2$, $t_3$ are provided.
Output: the risk class is provided: high, medium, low.

Method: for each risk factor $h_i = \lim_{\Delta t \to 0} \frac{E(t)/N(t)}{\Delta t}$, perform the following calculations:

1.
$$x_h[i] = \sup(h[i], D_p) - \sup(h[i], D_n);$$

2.
$$p = exp\left(\frac{\sum_{i=1}^{n} w_i ln x_i}{\sum_{i=1}^{n} w_i}\right)$$

3.
$$risk = (1 - y^p) * 100$$

4. if risk >=$t_3$, output risk = high
5. otherwise, if risk >=$t_2$, output risk = medium
6. otherwise, output risk = low.

Therefore, by means of the present invention, the evaluation of the risk for the onset of age-related macular degeneration (AMD) can be objectively estimated.

The test then provided an estimate of the risk of the subject to develop or not an Advanced Macular Degeneration (AMD) and it had to be interpreted in the clinical context and based on the lifestyle of the subject itself. Having a genetic predisposition to AMD does not necessarily mean that the disease will develop; vice versa, a low genetic risk does not guarantee to prevent the onset of AMD.

In general, a good mathematical model in a statistical test should be a good compromise between simplicity and accuracy, and this occurs because each model is nothing more than a more or less accurate approximation of reality. Therefore, any mathematical model is as accurate as the input parameters used for the mathematical representation thereof are consistent.

The present invention has been described herein with reference to the preferred embodiments thereof, but it will be understood that equivalent modifications may be made without departing from the scope of the protection afforded thereto.

Accordingly, the scope of protection of the present invention must not be limited to the particular embodiments described above by way of mere example, but must be considered according to the appended claims.

## Claims

1. A method for estimating the risk factor (HR) for the onset of macular degeneration in a patient, said method comprising the evaluation of a first series of genetic parameters which comprises at least the genetic loci R1210C in CFH, variants in COL8A1 and RAD51B, and the evaluation of a second series of ambient, individual and/or clinical parameters, wherein said risk factor (HR) is calculated by assigning a greater importance to said evaluation of the first series of genetic parameters with respect to said evaluation of the second series of ambient, individual and/or clinical parameters, **characterized in that** said risk factor (HR) is calculated by assigning an importance in a range between 51% and 70% to the evaluation of said first series of genetic parameters and an importance in a range between 30% and 49% to the evaluation of said second series of ambient, individual and/or clinical parameters.

2. A method according to claim 1, wherein said risk factor (HR) is calculated by assigning an importance of approximately 60% to the evaluation of said first series of genetic parameters and an importance of approximately 40% to the evaluation of said second series of ambient, individual and/or clinical parameters.

3. A method according to any one of the preceding claims, wherein said first series of genetic parameters further comprises at least the following additional genes: ARMS2 (Age-Related Maculopathy Susceptibility 2), CFH (Complement Factor H), C3 (Complement component 3); CFB (Complement factor B) and C2 (Complement Component 2).

4. A method according to claim 3, wherein said first series of genetic parameters further comprises at least the following polymorphisms of said additional genes: polymorphism rs10490924 of ARMS2; polymorphisms of rs1061170, rs1410996 and rs403846 of CFH; polymorphism rs2230199 of C3; polymorphism rs641153 of CFB and polymorphism rs9332739 of C2.

5. A method according to any one of the preceding claims, wherein said ambient parameters of said second series are selected from the group which comprises at least those relative to the fact that if the patient is a smoker, and those relative to an oxidant-based diet.

6. A method according to any one of the preceding claims, wherein said clinical parameters of said second series are selected from the group relative to cardiovascular problems, hypertension, diabetes, family history, AMD diagnosed in one eye and previous cataract operations.

7. A method according to any one of the preceding claims, wherein said individual parameters of said second series are selected from the group relative to age, gender and body mass index.

8. A method according to any one of the preceding claims, wherein the evaluation of said first series of genetic parameters is calculated by taking a mean of the individual evaluations obtained for each of said genetic parameters, each of said individual evaluations being obtained on the basis of the ratio between the events observed in a period of time ($\Delta t$) and the number of possible events at risk at the beginning of said period of time ($\Delta t$), when said time period ($\Delta t$) tends to zero; wherein the evaluation of said second series of ambient, individual and/or clinical parameters is calculated by taking a mean of the individual evaluations obtained for each of said ambient, individual and/or clinical parameters, each of said individual evaluations being obtained on the basis of the ratio between the events observed in a time period (Δt) and the number of possible events at risk at the beginning of said time period (Δt), when said time period (Δt) tends to zero.

9. A method according to any one of the preceding claims, wherein said risk factor (HR) is evaluated high, medium or low, according to whether by means of the method of the present invention it is respectively greater than given predefined threshold values t3, t2 or t1, with t3 > t2 > t1.

**Patentansprüche**

1. Verfahren zur Abschätzung des Risikofaktors (HR) für das Auftreten der Makuladegeneration bei einem Patienten, wobei das Verfahren die Bewertung einer ersten Reihe von genetischen Parametern aufweist, die zumindest die genetischen Loci R1210C in CFH, Varianten in COL8A1 und RAD51B aufweisen, sowie die Bewertung einer zweiten Reihe von Umgebungs-, Individual- und / oder klinischen Parametern, wobei der Risikofaktor (HR) errechnet wird, indem der Bewertung der ersten Reihe von genetischen Parametern gegenüber der Bewertung der zweiten Reihe von Umgebungs-, Individual- und / oder klinischen Parametern größere Bedeutung beigemessen wird, **dadurch gekennzeichnet, dass**
der Risikofaktor (HR) errechnet wird, indem der Bewertung der ersten Reihe von genetischen Parametern eine Gewichtung im Bereich zwischen 51% und 70% und der Bewertung der zweiten Reihe von Umgebungs-, Individual- und / oder klinischen Parametern eine Gewichtung im Bereich zwischen 30% und 49% beigemessen wird.

2. Verfahren gemäß Anspruch 1, wobei der Risikofaktor (HR) errechnet wird, indem der Bewertung der ersten Reihe von genetischen Parametern eine Gewichtung von etwa 60% und der Bewertung der zweiten Reihe von Umgebungs-, Individual- und / oder klinischen Parametern eine Gewichtung von etwa 40% beigemessen wird.

3. Verfahren gemäß einem der vorigen Ansprüche, wobei die erste Reihe von genetischen Parametern des Weiteren zumindest die folgenden weiteren Gene einbezieht: ARMS2 (altersbedingte Anfälligkeit für Makulopathie 2), CFH (Komplementärfaktor H), C3 (Komplementärkomponente 3), CFB (Komplementärfaktor B) und C2 (Komplementärkomponente 2).

4. Verfahren gemäß Anspruch 3, wobei die erste Reihe von genetischen Parametern des Weiteren zumindest die folgenden Polymorphismen der weiteren Gene einbezieht: Polymorphismus rs10490924 von ARMS2; Polymorphismen von rs1061170, rs1410996 und rs403846 von CFH; Polymorphismus rs2230199 von C3; Polymorphismus rs641153 von CFB und Polymorphismus rs9332739 von C2.

5. Verfahren gemäß einem der vorigen Ansprüche, wobei die Umgebungsparameter der zweiten Reihe ausgewählt werden aus der Gruppe, die zumindest den Fakt einbezieht, ob der Patient Raucher ist oder den Fakt einbezieht, ob eine oxidantienbasierte Diät vorliegt.

6. Verfahren gemäß einem der vorigen Ansprüche, wobei die klinischen Parameter der zweiten Reihe ausgewählt werden aus der Gruppe bezüglich Herz-Kreislaufproblemen, Bluthochdruck, Diabetes, Familiengeschichte, diagnostizierter AMD auf einem Auge und vorheriger Katarakt-Operationen.

7. Verfahren gemäß einem der vorigen Ansprüche, wobei die Individualparameter der zweiten Reihe ausgewählt werden aus der Gruppe bezüglich Alter, Geschlecht und Body-Mass-Index.

8. Verfahren gemäß einem der vorigen Ansprüche, wobei die Bewertung der ersten Reihe von genetischen Parametern berechnet wird, indem der Durchschnitt der Individualbewertungen genommen wird, die für jeden der genetischen Parameter gewonnen werden, und jede der Individualbewertungen auf Basis des Verhältnisses zwischen den in einem bestimmten Zeitraum (Δt) beobachteten Ereignissen und der Anzahl möglicher Risikoereignisse zu Beginn des Zeitraums (Δt), wenn der Zeitraum (Δt) gegen Null geht, gewonnen wird,
wobei die Bewertung der zweiten Reihe von Umgebungs-, Individual- und / oder klinischen Parametern berechnet wird, indem der Durchschnitt der Individualbewertungen genommen wird, die für jeden der Umgebungs-, Individual- und / oder klinischen Bewertungen Parameter gewonnen werden, und jede der Individualbewertungen auf Basis des Verhältnisses zwischen den in einem bestimmten Zeitraum (Δt) beobachteten Ereignissen und der Anzahl möglicher Risikoereignisse zu Beginn des Zeitraums (Δt), wenn der Zeitraum (Δt) gegen Null geht, gewonnen wird.

9. Verfahren gemäß einem der vorigen Ansprüche, wobei der Risikofaktor (HR) als hoch, mittel oder niedrig bewertet wird, je nachdem, ob er gemäß des Verfahrens der vorliegenden Erfindung jeweils größer ist als die vorgegebenen Schwellenwerte t3, t2 oder t1, wobei t3 > t2 > t1 ist.

## Revendications

1. Un procédé pour estimer le facteur de risque (HR) pour l'apparition d'une dégénérescence maculaire chez un patient, ledit procédé comprenant l'évaluation d'une première série de paramètres génétiques qui comprend au moins les loci génétiques R1210C dans CFH, des variants dans COL8A1 et RAD51B, et l'évaluation d'une deuxième série de paramètres ambiants, individuels et/ou cliniques, dans lequel ledit facteur de risque (HR) est calculé en attribuant une importance supérieure à ladite évaluation de la première série de paramètres génétiques par rapport à ladite évaluation de la deuxième série de paramètres ambiants, individuels et/ou cliniques, **caractérisé en ce que** ledit facteur de risque (HR) est calculé en attribuant une importance dans une plage comprise entre 51 % et 70 % à l'évaluation de ladite première série de paramètres génétiques et une importance dans une plage comprise entre 30 % et 49 % à l'évaluation de ladite deuxième série de paramètres ambiants, individuels et/ou cliniques.

2. Un procédé selon la revendication 1, dans lequel ledit facteur de risque (HR) est calculé en attribuant une importance d'approximativement 60 % à l'évaluation de ladite première série de paramètres génétiques et une importance d'approximativement 40 % à l'évaluation de ladite deuxième série de paramètres ambiants, individuels et/ou cliniques.

3. Un procédé selon l'une quelconque des revendications précédentes, dans lequel ladite première série de paramètres génétiques comprend en outre au moins les gènes supplémentaires suivants : ARMS2 (susceptibilité à la maculopathie liée à l'âge), CFH (facteur de complément H), C3 (composant de complément 3) ; CFB (facteur de complément B) et C2 (composant de complément 2).

4. Un procédé selon la revendication 3, dans lequel ladite première série de paramètres génétiques comprend en outre au moins les polymorphismes suivants desdits gènes supplémentaires : polymorphisme rs10490924 de ARMS2 ; polymorphismes de rs1061170, rs1410996 et rs403846 de CFH ; polymorphisme rs2230199 de C3 ; polymorphisme rs641153 de CFB et polymorphisme rs9332739 de C2.

5. Un procédé selon l'une quelconque des revendications précédentes, dans lequel lesdits paramètres ambiants de ladite deuxième série sont sélectionnés dans le groupe qui comprend au moins ceux relatifs au fait de savoir si le patient est un fumeur, et ceux relatifs à un régime à base d'oxydant.

6. Un procédé selon l'une quelconque des revendications précédentes, dans lequel lesdits paramètres cliniques de ladite deuxième série sont sélectionnés dans le groupe relatif aux problèmes cardiovasculaires, à l'hypertension, au diabète, à l'historique de la famille, à la DMA diagnostiquée dans un œil et à des opérations précédentes de la cataracte.

7. Un procédé selon l'une quelconque des revendications précédentes, dans lequel lesdits paramètres individuels de ladite deuxième série sont sélectionnés dans le groupe relatif à l'âge, au genre et à l'indice de masse corporelle.

8. Un procédé selon l'une quelconque des revendications précédentes, dans lequel l'évaluation de ladite première série de paramètres génétiques est calculée en prenant une moyenne des évaluations individuelles obtenues pour chacun desdits paramètres génétiques, chacune desdites évaluations individuelles étant obtenue sur la base du rapport entre les événements observés dans une période de temps ($\Delta t$) et le nombre d'événements possibles à risque au début de ladite période de temps ($\Delta t$), lorsque ladite période de temps ($\Delta t$) tend vers zéro ; dans lequel l'évaluation de ladite deuxième série de paramètres ambiants, individuels et/ou cliniques est calculée en prenant une moyenne des évaluations individuelles obtenues pour chacun desdits paramètres ambiants, individuels et/ou cliniques, chacune desdites évaluations individuelles étant obtenue sur la base du rapport entre les événements observés dans une période de temps ($\Delta t$) et le nombre d'événements possibles à risque au début de ladite période de temps ($\Delta t$), lorsque ladite période de temps ($\Delta t$) tend vers zéro.

9. Un procédé selon l'une quelconque des revendications précédentes, dans lequel ledit facteur de risque (HR) est évalué comme élevé, moyen ou faible, selon le fait de savoir si au moyen du procédé de la présente invention il est respectivement supérieur à des valeurs seuils prédéfinies t3, t2 ou t1, avec t3 > t2 > t1.